# EUROPEAN PATENT APPLICATION

(11) **EP 4 529 931 A1**
(43) Date of publication of application: **02.04.2025**
(21) Application number: 23842776.9
(22) Date of filing: 26.06.2023
(51) Int. Cl.: A61L 2/10, C02F 1/32

(54) **FLUID STERILIZATION DEVICE**

(30) Priority: 22.07.2022 JP 2022117218
(71) Applicant: Stanley Electric Co. Ltd., Tokyo 153-8636 (JP)
(72) Inventor: TANAKA, Hideaki, Tokyo 153-8636 (JP); KATO, Hiroyuki, Tokyo 153-8636 (JP); SHINNO, Kazuhisa, Tokyo 153-8636 (JP); KAWASAKI, Kazuaki, Tokyo 153-8636 (JP); SANO, Ryo, Tokyo 153-8636 (JP)
(74) Representative: Pritzlaff, Stefanie Lydia
(86) International application number: PCT/JP2023/023598
(87) International publication number: WO 2024/018845

(57) **Abstract**

A housing main body 12 and an outer cover 14 constitute a housing. A straight pipe 18 and a light source device 19 are inserted into the housing in an axial direction. An outlet flow path 35 is formed in the housing and communicates with a notch 181 formed on the light source device 19 side of the straight pipe 18. A shielding ring 20 has higher ultraviolet resistance than the housing. A tapered portion 201 of a shielding ring 20 defines an outlet flow path 35 and protects the housing from ultraviolet rays leaking out to an outside of the straight pipe 18 from the notch 181.

## Description

### Technical Field

The present invention relates to a fluid sterilization device that irradiates a fluid with ultraviolet rays to sterilize the fluid.

### Background Art

It is known that a sterilization target fluid, which flows in a flow path pipe from one end side to the other end side, is irradiated with ultraviolet rays from the other end side to be sterilized. In this case, in order to discharge the sterilization target fluid in the flow path pipe to the outside of the flow path pipe, an outlet port is provided on the other end side of the flow path pipe (for example, Patent Literature 1 and Patent Literature 2).

### Citation List

### Patent Literatures

Patent Literature 1: Japanese Patent Application Laid-Open No. 2018-202205
Patent Literature 2: Japanese Patent Application Laid-Open No. 2022-68062

### Summary of Invention

### Technical Problem

When the fluid sterilization device has a structure in which components such as a flow path pipe and a light source are inserted and stored as storage components from an opening side of the housing and are fastened from the opening side to be assembled, an assembly structure is simplified, and a disposition space is saved, and the size reduction can be achieved. In that case, typically, a flow path pipe made of a material having excellent ultraviolet resistance, such as polytetrafluoroethylene 4-fluorinated ethylene resin (PTFE), is used, while a housing made of a resin having rigidity and low cost is normally used.

In a case where the outlet port is provided at the other end portion of the flow path pipe, that is, the end portion on the light source side, there is a problem in that the ultraviolet intensity in the vicinity of the outlet port is high, and the housing receives ultraviolet rays leaking out from the outlet port to the outside of the flow path pipe and is likely to deteriorate.

The fluid sterilization devices of Patent Literature 1 and Patent Literature 2 do not have a structure in which the flow path pipe is stored in the housing together with other components such as a light source to rationalize assembly, and the problem is not raised that the housing deteriorates due to ultraviolet rays leaking out from the outlet port to the outside of the flow path pipe.

An object of the present invention is to provide a fluid sterilization device that can effectively prevent deterioration of a housing caused by ultraviolet rays leaking out from an outlet port to an outside of a flow path pipe.

### Solution to Problem

According to an aspect of the present invention, there is provided a fluid sterilization device that sterilizes a liquid, the device including:
a housing configured to have one end side and the other end side on a coaxial axis as a straight line,
in which the housing includes a first tubular portion on the one end side and a second tubular portion on the other end side having a larger inner diameter than the first tubular portion;
a flow path pipe configured to be inserted into the first tubular portion of the housing in an axial direction such that a sterilization target fluid flows from the one end side to the other end side in one direction, and to have a notch extending in the axial direction by a predetermined length from an opening on the other end side toward the one end side;
an opening blocking member configured to be provided in the second tubular portion of the housing, to include an ultraviolet transmitting portion, and to block the opening of the flow path pipe;
a light source configured to irradiate an inside of the flow path pipe with ultraviolet rays from the other end side in the axial direction through the ultraviolet transmitting portion of the opening blocking member; and
a shielding member configured to be provided in the second tubular portion of the housing, to have higher ultraviolet resistance than the housing, to be disposed on an outer peripheral side of the flow path pipe, to define an upstream end portion of an outlet flow path of the sterilization target fluid from the notch, and to shield an inner peripheral side of the housing from emitted light of ultraviolet rays from the notch of the flow path pipe.

### Effect of the Invention

According to the present invention, the shielding member having higher ultraviolet resistance than the housing is provided in an irradiation range of ultraviolet rays leaking out from the notch of the other end portion of the flow path pipe to the outside, and the housing is shielded from the leaked ultraviolet rays. Accordingly, it is possible to reduce the size of the fluid sterilization device while protecting the housing from ultraviolet rays.

### Brief Description of Drawings

FIG. 1 is a longitudinal cross-sectional view of a fluid sterilization device.
FIG. 2 is an exploded perspective view illustrating a storage component stored in a cylindrical portion of a housing main body disassembled in an axial direction of the fluid sterilization device.
FIG. 3 is an exploded perspective view when a light source device and an outlet are disassembled in the axial direction and viewed from one end side in the axial direction.
FIG. 4 is an exploded perspective view when the light source device and the outlet are disassembled in the axial direction and viewed from the other end side in the axial direction.
FIG. 5A is an enlarged view of a range including an expanded diameter portion and an outer cover in the axial direction in FIG. 1.
FIG. 5B is an enlarged view of an upper half portion with respect to a central axis Rx in FIG. 5A.
FIG. 6 is a perspective view of a reflector according to a modification example.
FIG. 7 is a diagram in which an illuminance distribution of UV rays in the fluid sterilization device is analyzed by simulation with an upper limit of the illuminance distribution of 40 mw/cm².
FIG. 8 is a diagram in which an illuminance distribution of UV rays in the fluid sterilization device is analyzed by simulation with an upper limit of the illuminance distribution of 10 mw/cm².
FIG. 9 is a diagram in which an illuminance distribution of UV rays in the fluid sterilization device is analyzed by simulation with an upper limit of the illuminance distribution of 5 mw/cm².

### Description of Embodiments

Hereinafter, an embodiment of the present invention will be described. It goes without saying that the present invention is not limited to the embodiment. The components common to a plurality of embodiments are denoted by the same reference numerals throughout all the drawings.

### (Configuration)

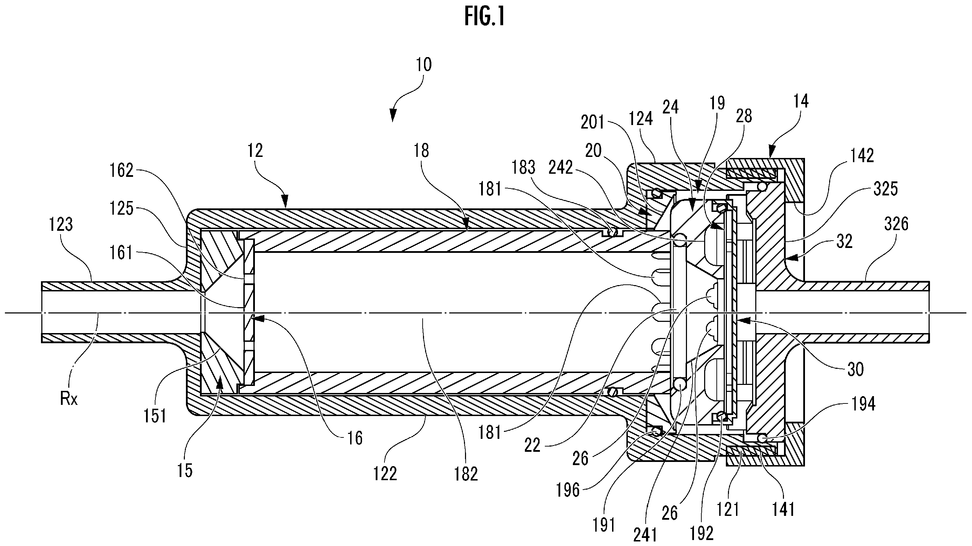

FIG. 1 is a longitudinal cross-sectional view of a fluid sterilization device 10. Rx illustrates a central axis of the fluid sterilization device 10. The fluid sterilization device 10 has a cylindrical housing main body 12 and a cap-shaped outer cover 14. The housing main body 12 and the outer cover 14 constitute a housing of the fluid sterilization device 10, and are aligned with a central axis of the fluid sterilization device 10 with the central axis Rx and are screwed into a male screw portion 121 and a female screw portion 141, respectively. The male screw portion 121 and the female screw portion 141 are screwed with each other by fastening the storage component in the housing in the axial direction, thereby simplifying an assembly structure of the storage component.

The housing main body 12 has a cylindrical portion 122, an inlet 123, an expanded diameter portion 124, and a stopper portion 125. The inlet 123 protrudes from one end side in the axial direction of the cylindrical portion 122 toward the one end side along the central axis Rx by a predetermined length. The stopper portion 125 is formed as an inner surface of the cylindrical portion 122 on one end side in the axial direction, and an inlet passage of the inlet 123 is open at the center portion. The stopper portion 125 has the role of preventing the storage component in the cylindrical portion 122 from moving to one end side in the axial direction. The expanded diameter portion 124 protrudes from the cylindrical portion 122 in the radial direction on one end side and is open on the other end side.

The outer cover 14 has an opening 142 at the center of the cover portion. The outer cover 14 has the role of preventing the storage component from being separated from the housing main body 12 by a cover portion that defines a circular opening 142 in the central portion, and pressing the storage component toward the stopper portion 125 after screwing with the housing main body 12.

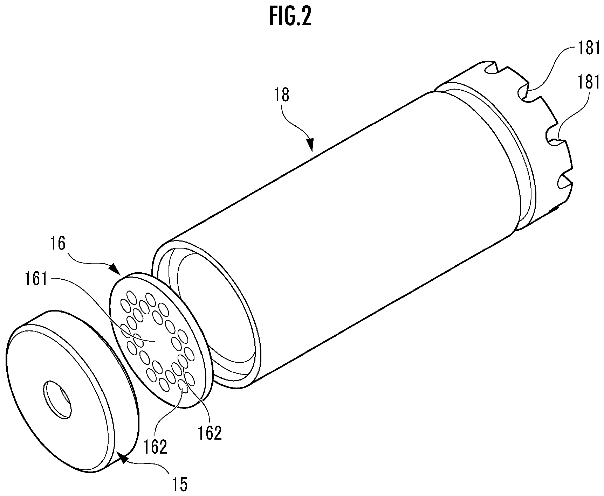

FIG. 2 is an exploded perspective view illustrating the storage component stored in the cylindrical portion 122 of the housing main body 12 disassembled in an axial direction of the fluid sterilization device 10. The storage components include a shielding body 15, a rectifying plate 16, and a straight pipe 18, the central axes thereof are aligned with the central axis Rx of the housing, which are arranged in the order from one end side to the other end side in the axial direction, and inserted from the opening side of the other end side of the housing main body 12.

The shielding body 15, the rectifying plate 16, and the straight pipe 18 are made of a material having ultraviolet resistance. The one end side of the shielding body 15 is brought into contact with an inner surface of the stopper portion 125, and the inner surface of the stopper portion 125 is shielded from ultraviolet (UV) rays to protect the stopper portion 125 from UV rays. The shielding body 15 also includes a tapered portion 151 on the inner peripheral side. The tapered portion 151 has a diameter equal to the inner diameters of the inlet 123 and the straight pipe 18 on each of the small diameter side and the large diameter side, and communicates with each of the inlet 123 and a sterilization chamber 182. By having the tapered portion 151, the UV rays reflected by the tapered portion 151 are reflected in the direction of the rectifying plate 16, and thus the utilization efficiency of the UV rays is increased.

The rectifying plate 16 has a plurality of rectifying holes 162 in a peripheral portion surrounding a central portion 161. The central portion 161 functions as a dam for a sterilization target fluid (for example, water) that flows into the rectifying plate 16 from a pressure feed pump (not illustrated) through the inlet 123 and the shielding body 15. That is, the sterilization target fluid is brought into contact with the central portion 161, is decelerated, and then flows into the straight pipe 18 from the rectifying hole 162. As a result, the flow velocity of the sterilization target fluid is uniformed inside and outside the sterilization chamber 182 in the radial direction. In addition, since an inlet port of the shielding body 15 (the minimum diameter portion of the tapered portion 151) and the central portion 161 of the rectifying plate 16 are provided on the central axis Rx, the UV rays emitted from the straight pipe 18 in the rectifying plate direction is prevented from leaking from the fluid sterilization device 10. Here, the area of the central portion 161 is equal to or larger than the area of the inlet port of the shielding body 15.

The straight pipe 18 defines the sterilization chamber 182 on the inner peripheral side. A plurality of notches 181 is formed in a peripheral wall of the straight pipe 18 on the other end side in the axial direction at equal angular intervals in the circumferential direction, and extends from the opening at the other end of the straight pipe 18 to the one end side by a predetermined length. An O-ring 183 is fitted to an annular groove of the outer peripheral portion of the straight pipe 18 and prevents the leakage of the sterilization target fluid at the outer peripheral portion.

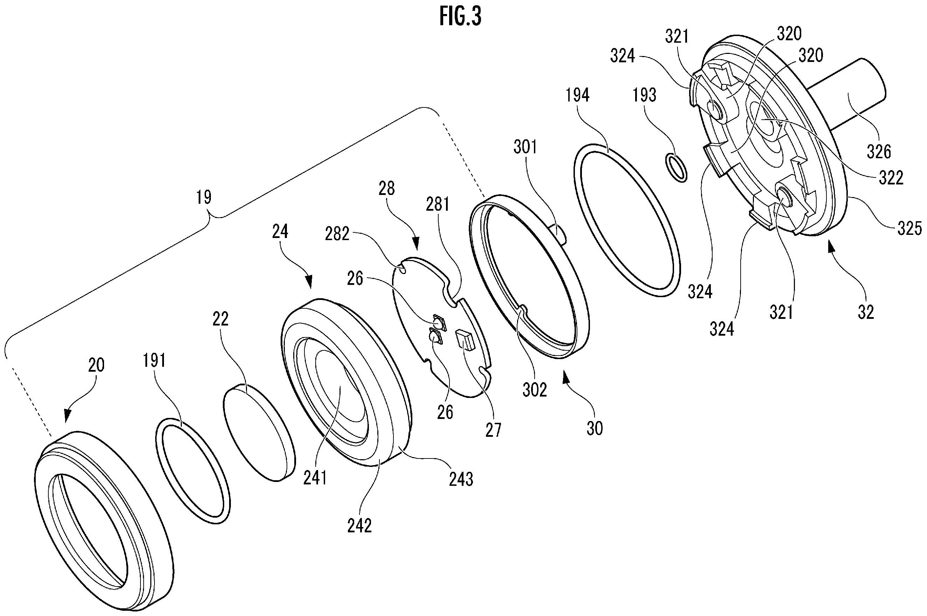

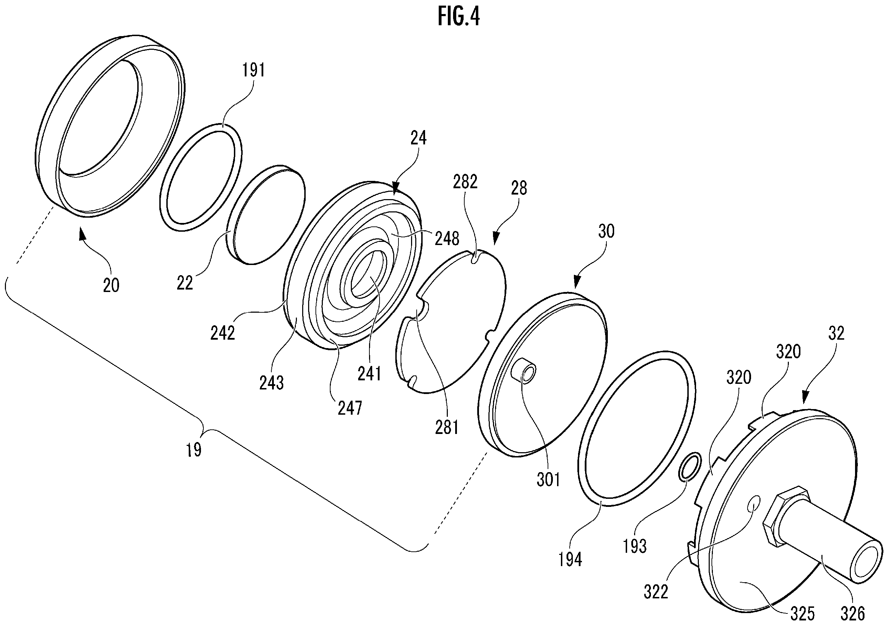

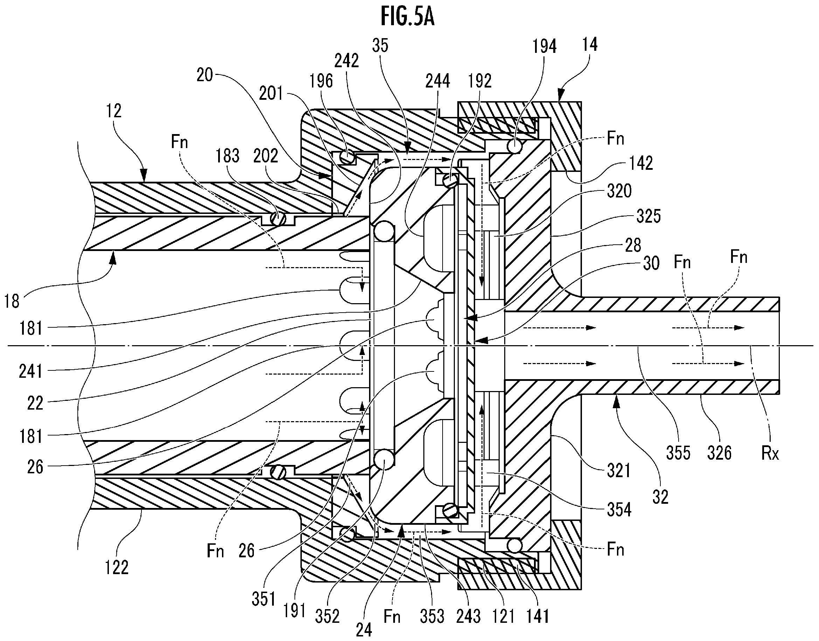

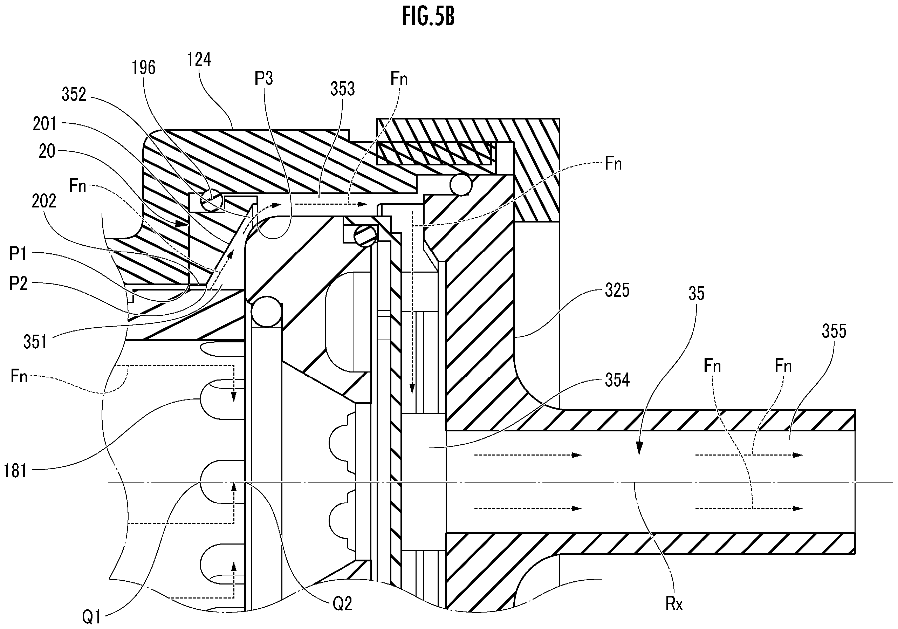

FIGS. 3 and 4 are exploded perspective views when a light source device 19 and a housing blocking member 32 are disassembled in the axial direction and viewed from one end side and the other end side in the axial direction, respectively. FIG. 5A is an enlarged view of a range including the expanded diameter portion 124 and the outer cover 14 in the axial direction in FIG. 1, and FIG. 5B is an enlarged view of an upper half portion with respect to the central axis Rx in FIG. 5A.

In FIGS. 3 to 5B, the light source device 19 and the housing blocking member 32 are stored in the expanded diameter portion 124 in an arrangement of one end side and the other end side, respectively, and with the central axes thereof aligned with the central axis Rx. The light source device 19 is disassembled into a shielding ring 20, an O-ring 191, a quartz glass 22, a reflector 24, a UV-LED 26, a substrate 28, and a heat radiation cover 30 in order from one end side to the other end side in the axial direction.

In the light source device 19, a side on which the UV rays are emitted and a side opposite to the side thereof are appropriately referred to as a front surface side and a rear surface side, respectively. The light source device 19 is disposed such that each of the front surface side and the rear surface side are directed to one end side and the other end side of the fluid sterilization device 10 in the axial direction. The O-ring 191 is fitted between a peripheral portion of the quartz glass 22 and an annular step portion on the front surface side of the reflector 24 and sealing is performed.

The quartz glass 22 and the O-ring 191 constitute an opening blocking member that blocks the other end-side opening of the straight pipe 18 as the flow path pipe.

The reflector 24 and the shielding ring 20 are made of a material having ultraviolet resistance. The reflector 24 has a reflecting surface 241, a protruding surface 242, a peripheral surface 243, and a recess 244. The reflecting surface 241 is formed in a tapered shape in which a diameter gradually increases from the rear surface side toward the front surface side on an inner peripheral side of the reflector 24. The protruding surface 242 protrudes from the peripheral edge of the reflecting surface 241 to the outer side in the radial direction. The peripheral surface 243 is formed in the shape of a columnar side surface and extends from the outer end in the radial direction of the protruding surface 242 to the other end in the axial direction. The recess 244 is formed by being opened in a peripheral portion on the rear surface of the reflector 24.

The reflector 24 has a circumferential end surface 247 and an annular step portion 248 formed along an inner periphery of the circumferential end surface 247 and recessed toward the rear on the rear surface side (FIG. 4).

A plurality (two in the illustrated example) of UV-LEDs 26 and a plurality of electrical components 27 are mounted on a center portion and a peripheral portion on the front surface side of the substrate 28, respectively. The UV-LED 26 is exposed in a tapered reflecting surface 241 from a rear surface side of the reflector 24, and the electrical component 27 is stored in the recess 244 on the rear surface of the reflector 24. A pair of recesses 281 and a pair of recesses 282 facing each other are formed on a peripheral edge of the circular substrate 28.

The UV rays emitted by the UV-LED 26 belong to deep ultraviolet rays having a high effect on sterilization of the fluid, and have a wavelength range of, for example, 100 to 400 nm. In particular, among the ultraviolet wavelength range, UV having a wavelength of 100 to 280 nm is further preferable since UV has a particularly high sterilization effect.

The heat radiation cover 30 is made of metal and has a tubular portion 301 on the rear surface side. A harness (not illustrated), which is a wiring of the substrate 28 to the electrical component 27, is inserted into the tubular portion 301. A pair of protrusions 302 is formed on the front surface side of the heat radiation cover 30 and is fitted into the pair of recesses 282 of the substrate 28. The O-ring 192 (FIG. 5A) is fitted between the inner periphery of the heat radiation cover 30 and the outer periphery of the annular step portion on the other end side in the axial direction of the peripheral surface 243.

The housing blocking member 32 includes a plurality of spacers 320 as the ridges formed at equal angular intervals in the circumferential direction on the inner surface side, and a projecting portion 321 formed on the ridge top surface of the spacer 320 in a relationship of being separated by 180° in the circumferential direction. An arc-shaped protruding edge 324 is fitted to the outside of the peripheral surface 243 of the reflector 24.

A harness hole 322 penetrates the housing blocking member 32 in the axial direction. The tubular portion 301 in which the O-ring 193 is fitted to the peripheral portion is fitted to the harness hole 322. The projecting portion 321 is formed for the purpose of sharing components with another type of fluid sterilization device, and can be omitted in the fluid sterilization device 10. This is because the positioning of the heat radiation cover 30 and the housing blocking member 32 in the circumferential direction is achieved by the fitting of the tubular portion 301 and the harness hole 322.

The housing blocking member 32 includes a blocking portion 325 and an outlet 326 that protrudes from the blocking portion 325 to the other end side in the axial direction along the central axis Rx on the rear surface side. The outlet 326 passes through the inner peripheral side of the opening 142 of the outer cover 14 and reaches the outside of the outer cover 14 at the protruding end.

In FIGS. 5A and 5B, in the shielding ring 20, an annular end surface on one end side in the axial direction is fitted to the inner periphery of the expanded diameter portion 124 by using an O-ring 196. The shielding ring 20 includes a columnar side surface portion 202 and a tapered portion 201 on the inner peripheral side of each of one end side and the other end side in the axial direction. In the illustrated example, in the axial direction, a position P1 at the end of the shielding ring 20 on one end side, a position P2 at the end of the tapered portion 201 on the small diameter side, a position P3 at the end of the tapered portion 201 on the large diameter side, a position Q1 at the terminal end of the notch 181 (the position at one end in the axial direction), and a starting end position of the notch 181 (the position at the other end in the axial direction), that is, the other end position Q2 of the straight pipe 18 are disposed as P1, Q1, P2, and P3 (= Q2) in order from one end side to the other end side in the axial direction. However, it is preferable that P1 and Q1 are at the same position in the axial direction (P1 = Q1). This is because the entire notch 181 is exposed to the tapered portion 201, the effective area of the notch 181 is increased, a space having a triangular cross-section is formed between one end side of the tapered portion 201 and the outer peripheral surface of the straight pipe 18, and the sterilization target fluid can be prevented from being retained in the triangular space.

In FIGS. 5A and 5B, Fn indicates a flow of the sterilization target fluid in the fluid sterilization device 10. An outlet flow path 35 is formed in a space between the light source device 19 in the fluid sterilization device 10 and the inner surface of the tapered portion 201 of the shielding ring 20, the expanded diameter portion 124, or the inner surface of the blocking portion 325 of the housing blocking member 32, as a passage for discharging the sterilization target fluid, which is discharged from the notch 181 of the straight pipe 18 to the outside in the radial direction, to the outside of the fluid sterilization device 10. The outlet flow path 35 includes a first flow path portion 351, a second flow path portion 352, a third flow path portion 353, a fourth flow path portion 354, and a fifth flow path portion 355 in order in the flow direction of the sterilization target fluid. The first flow path portion 351, the second flow path portion 352, and the third flow path portion 353 all have an annular shape when viewed in the axial direction. The fourth flow path portion 354 and the fifth flow path portion 355 have a circular shape when viewed in the axial direction.

The first flow path portion 351 is formed in a space interposed between the tapered portion 201 and the protruding surface 242 in the axial direction, and guides the sterilization target fluid immediately after being discharged from the notch 181 as the most upstream portion of the outlet flow path 35 to the downstream side. The second flow path portion 352 is formed as a passing portion between a corner portion of the boundary between the protruding surface 242 and the peripheral surface 243 and the tapered portion 201. The third flow path portion 353 is formed in an annular shape between the expanded diameter portion 124 and the peripheral surface 243. The fourth flow path portion 354 is formed as a gap in the axial direction between the rear surface of the light source device 19 and the inner surface of the housing blocking member 32.

### (Material)

The following is an example of a material of each component constituting the fluid sterilization device 10.
(a) Housing (housing main body 12 and outer cover 14): engineering plastics such as polycarbonate (PC) and polyacetal (POM)
(b) Shielding body 15, rectifying plate 16, straight pipe 18, shielding ring 20, and reflector 24: a sterilization target fluid such as polytetrafluoroethylene 4-fluorinated ethylene resin (PTFE), perfluoroalkoxyalkane (PFA), or polyvinyl fluoride (PVF), and polyvinylidene fluoride (PVDF), and a fluororesin
(c) Heat Radiation Cover 30: Metal

The material of (b) is selected as a material having ultraviolet resistance and reflectance to the UV rays higher than those of the material of (a). The material of (a) is selected as a material having higher anti-corrosion properties against the sterilization target fluid than the metal. The reason why the material of (b) is selected for the shielding body 15 and the shielding ring 20 is that the shielding body 15 and the shielding ring 20 are made of a member obtained by subjecting PTFE to mild processing, and thus, the materials can be manufactured by simple processing.

### (Action)

The sterilization target fluid is pumped from a pressure feed pump (not illustrated) to the fluid sterilization device 10 and introduced into the sterilization chamber 182 of the straight pipe 18 through the inlet 123 of the housing main body 12, a tapered portion 151 of the shielding body 15, and a rectifying hole 162 of the rectifying plate 16. The rectifying hole 162 is not provided in a central portion 161 of the rectifying plate 16. The target fluid collided with the central portion 161 before passing through the rectifying hole 162. As a result of this, the flow of target fluid in the sterilization chamber 182 is rectified and equalized in the speed of the central and peripheral portions of the chamber 182.

The UV-LED 26 emits the UV rays in a direction toward the quartz glass 22 in the axial direction of the light source device 19. Among ultraviolet rays emitted from the UV-LED 26, ultraviolet rays that spread in the radial direction and are emitted to the reflecting surface 241 are reflected by the reflecting surface 241 and are reflected toward the central axis Rx. Ultraviolet rays pass through the quartz glass 22 and are emitted to the sterilization target fluid in the sterilization chamber 182. As a result, the sterilization target fluid is sterilized.

The sterilization target fluid collides with the surface of the quartz glass 22, and thus the direction is changed from the axial direction of the straight pipe 18 to the outward direction in the radial direction, and the sterilization target fluid comes out of the straight pipe 18 from the notch 181. Since the total flow cross-sectional area of the plurality of notches 181 is smaller than the flow cross-sectional area of the sterilization chamber 182, the flow velocity of the sterilization target fluid increases in the notch 181. The flow velocity of the sterilization target fluid is further increased by the tapered portion 201.

In FIG. 1, the fluid sterilization device 10 is placed in a horizontally placed manner (a method of placing such that the longitudinal direction is aligned with the horizontal direction), but for example, can be used in a vertically placed manner (a method of placing such that the longitudinal direction is aligned with the vertical direction) in which each of the inlet 123 and the outlet 326 are at the bottom and the top. In this case, as the equipped device such as a water server provided with the fluid sterilization device 10 is stopped, the operation of the pressure feed pump is also stopped, and air remains in the upper portion of the straight pipe 18. It is preferable that the residual air is quickly discharged to the outside when the operation of the pressure feed pump is started next. This is because the air causes the intensity of the UV rays to be weakened.

As described above, since the flow velocity of the sterilization target fluid increases in the notch 181, in the fluid sterilization device 10, the air remaining in the upper portion of the straight pipe 18, that is, the height of the notch 181 is quickly and smoothly discharged to the outside of the straight pipe 18 by the sterilization target fluid at a high speed without remaining for a long time.

On the other hand, among the UV rays emitted from the quartz glass 22 to the sterilization chamber 182, the UV rays that are significantly spread outward in the radial direction are emitted from the notch 181 to the outside of the straight pipe 18. Hereinafter, the UV rays that enter the notch 181 from the inner peripheral side of the straight pipe 18 are also referred to as "externally leaked UV rays".

As described above, in the axial direction, the positional relationship in the axial direction between a position P1 of the shielding ring 20 on one end side, a position P2 of the boundary between the columnar side surface portion 202 and the tapered portion 201, a position P3 of the opening end of the shielding ring 20, a position Q1 of the terminal end of the notch 181, a starting end position of the notch 181, that is, the other end position Q2 of the straight pipe 18 is defined as described above. As a result, the externally leaked UV rays are shielded by the fluid sterilization device 10, and the irradiation of the inner surface of the housing main body 12 is prevented. That is, the entire amount of the externally leaked UV rays is radiated to the tapered portion 201 of the shielding ring 20, and is reflected to the inner side in the radial direction, or is reflected to the columnar side surface portion 202 via a part of the notch 181 on one end side and is immediately returned to the straight pipe 18. The remaining amount is radiated to the tapered portion 201 of the shielding ring 20 and is reflected.

On the other hand, the sterilization target fluid passes through the first flow path portion 351, and then passes through the second flow path portion 352 sandwiched between the tapered portion 201 and the corner portion of the reflector 24. The UV rays radiated to the tapered portion 201 among the externally leaked UV rays are reflected by the tapered portion 201 and then the reflection destination is divided into three portions of (a) the notch 181 in a peripheral wall of the other end portion of the straight pipe 18 in the axial direction, (b) a portion of the peripheral wall of the other end portion of the straight pipe 18 in the axial direction where the notch 181 is not formed, and (c) the protruding surface 242. Among the externally leaked UV rays, the UV rays of which the reflection destination is (a) pass through the notch 181 and return to the straight pipe 18, and contribute to the re-sterilization of the sterilization target fluid of the sterilization chamber 182. Among the externally leaked UV rays, the UV rays of which the reflection destination is (b) and (c) are reflected again on the reflection destination, are repeatedly reflected between the tapered portion 201 and the reflector 24 until the intensity is sufficiently weakened, and contribute to the sterilization of the sterilization target fluid in the first flow path portion 351 and the second flow path portion 352.

The tapered portion 201 is set to have a value of a taper angle or a contour shape such that the externally leaked UV rays remain in the first flow path portion 351 and the second flow path portion 352 without leaking to the third flow path portion 353 on the downstream side of the second flow path portion 352 due to the alternated and repeated reflection between the tapered portion 201 and the reflector 24, or the externally leaked UV rays finally return to the sterilization chamber 182 via the notch 181.

After passing through the second flow path portion 352, the sterilization target fluid flows in the annular third flow path portion 353 between the peripheral surface 243 of the reflector 24 and the inner peripheral surface of the expanded diameter portion 124 in the axial direction, and then comes into contact with the inner surface of the blocking portion 325 to change the traveling direction to the inner side in the radial direction. The sterilization target fluid flows around into the fourth flow path portion 354 on the rear surface side of the light source device 19 and is collected at the opening on one end side of the outlet 326 as a center portion in the radial direction along the inner surface of the blocking portion 325. The fourth flow path portion 354 is formed as a gap interposed between the rear surface of the light source device 19 and the blocking portion 325 of the housing blocking member 32 in the axial direction.

The sterilization target fluid comes into contact with the rear surface of the heat radiation cover 30 of the light source device 19 in the fourth flow path portion 354 to cool the heat radiation cover 30. Since the heat generated by the UV-LED 26 is conducted to the substrate 28 and further conducted to the metal heat radiation cover 30, the cooling of the heat radiation cover 30 by the sterilization target fluid in the fourth flow path portion 354 contributes to the cooling of the UV-LED 26. Here, the flow velocity of the sterilization target fluid is increased by the notch 181 and the tapered portion 201, and thus the cooling property of the heat radiation cover 30 is improved.

The substrate 28 is also referred to as a metal substrate, a mounting region for a component requiring heat radiation is made of metal, and has a structure in which the thermal conductivity to a rear surface side is increased.

The sterilization target fluid flows out to the outside of the fluid sterilization device 10 through the fifth flow path portion 355.

### (Modification Example of Reflector)

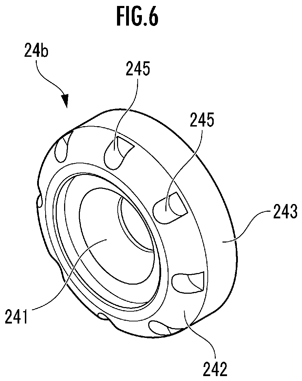

FIG. 6 is a perspective view of a reflector 24b according to a modification example. The reflector 24b differs from the reflector 24 (FIGS. 3 and 4) in that the protruding surface 242 is not perpendicular to the central axis and is formed in a tapered shape that matches the shape of the tapered portion 201, and a plurality of grooves 245 is formed in the tapered protruding surface 242 at equal angular intervals in the circumferential direction.

As a result, the protruding surface 242 of the reflector 24b can prevent the interval between the protruding surface 242 of the reflector 24 and the tapered portion 201 from being locally narrowed. In addition, the groove 245 can ensure a sufficiently large flow cross-sectional area of the first flow path portion 351.

### (Analysis of Illuminance Distribution)

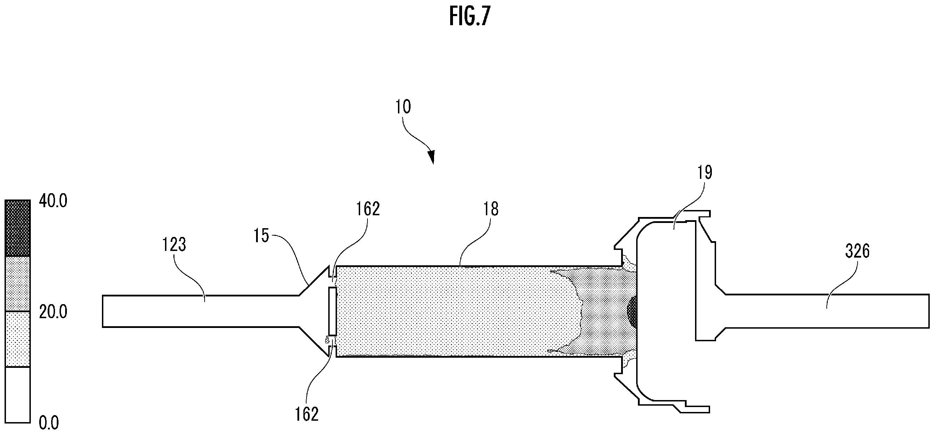

FIGS. 7 to 9 are diagrams in which an illuminance distribution of UV rays in the fluid sterilization device 10 is analyzed by simulation. In the analysis of the illuminance distribution in FIGS. 7 to 9, the emission intensity (brightness) of the UV rays emitted from the UV-LED 26 is the same. However, the upper limit of the illuminance distribution analysis is set to 40 mw/cm², 10 mw/cm², and 5 mw/cm², respectively. Therefore, in each illuminance distribution diagram, positions of the illuminance equal to or higher than the upper limit are uniformly assigned to the highest stage region. The simulation was performed using ASAP of Breault Research Organization.

In addition, in the description of FIGS. 1 and 2, the straight pipe 18 side of the central portion 161 of the rectifying plate 16 is described as a reflecting surface of the UV rays, and in the illuminance distribution of FIGS. 7 to 9, it is calculated that the UV rays are not reflected at the central portion 161.

In the illuminance distributions of FIGS. 7 to 9, the upper limit illuminance Lu is divided into four equal parts, and it is illustrated which stage of the four illuminance stages each position belongs to. That is, when the illuminance is L, the first illuminance stage is 0 ≤ L < Lu/4, the second illuminance stage is Lu/4 ≤ L < Lu/2, the third illuminance stage is Lu/2 ≤ L < 3 x Lu/4, and the fourth illuminance stage is 3 x Lu/4 ≤ L.

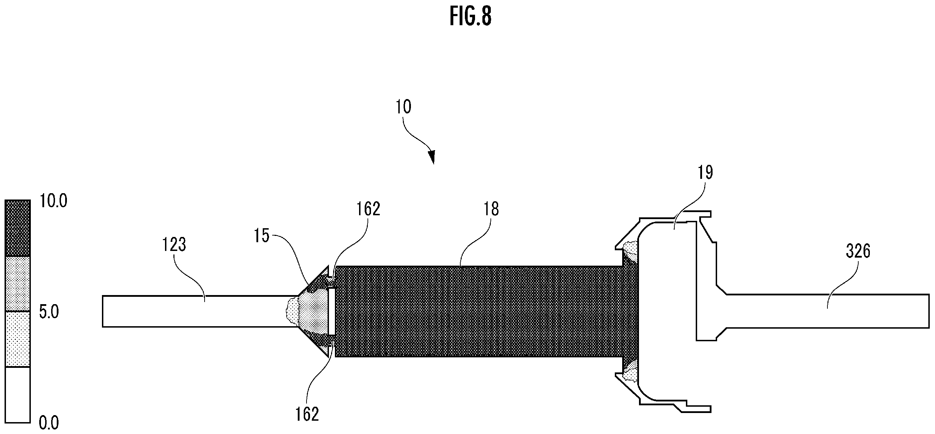

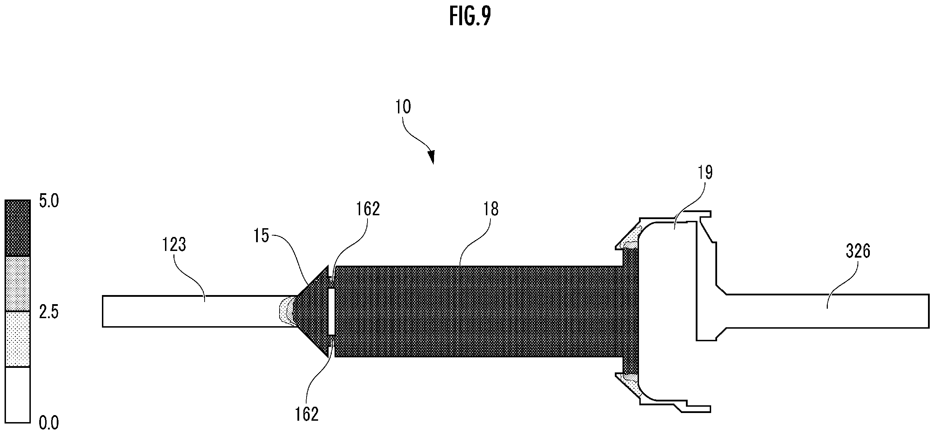

From FIG. 7, it can be seen that the emission vicinity of the UV-LED 26 has a strong illuminance of 30 mw/cm² or more, and there is an illuminance region of 10 mw/cm² to 20 mw on the outer peripheral side of the notch 181. From FIG. 8, it can be seen that the inner side portion in the radial direction of the tapered portion 201 and the shielding body 15 are in an illuminance region of 2.5 mw/cm² to 5.0 mw, and from FIG. 9, it can be seen that the maximum diameter side of the tapered portion 201 is in an illuminance region of 1.25 mw/cm² to 2.5 mw.

That is, from the simulation results of the illuminance distribution in FIGS. 7 to 9, by providing the tapered portion 201, it is possible to prevent ultraviolet light from leaking into the region of the housing (the housing main body 12 and the outer cover 14) and to prevent deterioration due to ultraviolet rays.

### (Supplement)

In the fluid sterilization device 10, water is used as the sterilization target fluid. However, in the present invention, the sterilization target fluid may be a liquid other than water.

The fluid sterilization device 10 is provided with two UV-LEDs 26 as light sources that emit the UV rays. In the fluid sterilization device according to the present invention, the number of light sources that emit the UV rays may be one, or three or more.

In the fluid sterilization device 10, the quartz glass 22 is used as the ultraviolet transmitting portion. As long as the ultraviolet transmitting portion according to the present invention transmits ultraviolet rays and has anti-corrosion properties with respect to the sterilization target fluid, a component other than the quartz glass 22 can be used.

The cylindrical portion 122 and the expanded diameter portion 124 of the fluid sterilization device 10 correspond to each of a first tubular portion and a second tubular portion according to the present invention, and store each of the straight pipe 18 as a flow path pipe and the light source device 19. The shielding ring 20 is fitted onto an end portion of the straight pipe 18 on the notch 181 side in the expanded diameter portion 124. The tapered portion 201 of the shielding ring 20 forms an outlet passage of the sterilization target fluid discharged to the outside from the notch 181 as the outlet port of the straight pipe 18 in the expanded diameter portion 124, and receives ultraviolet rays leaking out from the notch 181 to the outside of the straight pipe 18 to protect the expanded diameter portion 124.

Examples of a sterilization target fluid as a sterilization target by the fluid sterilization device 10 include water stored in a water tank of an ice maker, water supplied in a water supply pipe or a hot water heater, drinking water in a water server, cooling water in a circulation device (chiller), and a beverage liquid in a drink server.

### Description of Reference Numerals

- 10:: fluid sterilization device
- 18:: straight pipe (flow path pipe)
- 19:: light source device
- 20:: shielding ring (shielding member)
- 22:: quartz glass (ultraviolet transmitting portion)
- 24, 24b:: reflector
- 26:: UV-LED (light source)
- 28:: substrate
- 30:: heat radiation cover
- 32:: housing blocking member
- 35:: outlet flow path
- 122:: cylindrical portion (first tubular portion)
- 124:: expanded diameter portion (second tubular portion)
- 114:: outer cover
- 125:: stopper portion
- 142:: opening
- 181:: notch
- 201:: tapered portion
- 202:: columnar side surface portion
- 241:: reflecting surface
- 242:: protruding surface
- 243:: peripheral surface
- 245:: groove
- 325:: blocking portion
- 326:: outlet
- 351:: first flow path portion
- 352:: second flow path portion
- 353:: third flow path portion
- 354:: fourth flow path portion
- 355:: fifth flow path portion

## Claims

1. A fluid sterilization device that sterilizes a liquid, comprising:
a housing configured to have one end side and the other end side on a coaxial axis as a straight line,
in which the housing includes a first tubular portion on the one end side and a second tubular portion on the other end side having a larger inner diameter than the first tubular portion;
a flow path pipe configured to be inserted into the first tubular portion of the housing in an axial direction such that a sterilization target fluid flows from the one end side to the other end side in one direction, and to have a notch extending in the axial direction by a predetermined length from an opening on the other end side toward the one end side;
an opening blocking member configured to be provided in the second tubular portion of the housing, to include an ultraviolet transmitting portion, and to block the opening of the flow path pipe;
a light source configured to irradiate an inside of the flow path pipe with ultraviolet rays from the other end side in the axial direction through the ultraviolet transmitting portion of the opening blocking member; and
a shielding member configured to be provided in the second tubular portion of the housing, to have higher ultraviolet resistance than the housing, to be disposed on an outer peripheral side of the flow path pipe, to define an upstream end portion of an outlet flow path of the sterilization target fluid from the notch, and to shield an inner peripheral side of the housing from emitted light of ultraviolet rays from the notch of the flow path pipe.

2. The fluid sterilization device according to Claim 1, wherein
the shielding member includes a tapered portion configured to expand from the one end side toward the other end side in the axial direction of the flow path pipe on an inner peripheral side.

3. The fluid sterilization device according to Claim 2, wherein
the shielding member has a higher reflectance to ultraviolet rays than the housing.

4. The fluid sterilization device according to Claim 3, further comprising:
a reflector configured to be disposed between the light source and the opening blocking member in the axial direction, to have a reflecting surface reflecting ultraviolet rays from the light source on an inner peripheral side, and to form the outlet flow path in an annular gap between the shielding member and the housing on an outer peripheral side.

5. The fluid sterilization device according to Claim 4, wherein
the reflector has a protruding surface configured to protrude on an outside of the flow path pipe in a radial direction and to face the tapered portion in the axial direction, and has higher ultraviolet resistance than the housing, and
the tapered portion of the shielding member reflects ultraviolet rays toward the notch and the protruding surface of the reflector.

6. The fluid sterilization device according to Claim 4, wherein
the reflector includes a groove configured to extend along a flow of the sterilization target fluid in a portion defining the outlet flow path.

7. The fluid sterilization device according to Claim 4, wherein
the opening blocking member, the reflector, and the light source constitute a light source device,
the fluid sterilization device further comprises
a housing blocking member configured to include a blocking portion formed as a gap in the axial direction between the blocking portion and the light source device and an outlet configured to protrude from a center portion of the blocking portion toward an outside of the housing and to guide the sterilization target fluid in the gap to the outside of the housing,
the housing includes a stopper portion configured to prevent a movement of a storage component in the housing toward the one end side and a fastening portion configured to fasten the storage component toward the stopper portion through the blocking portion of the housing blocking member on the one end side in the axial direction,
the flow path pipe and the light source device are stored in the housing as the storage component, and
the flow path pipe, the light source device, and the housing blocking member are arranged in a row in the axial direction in order from the one end side with central axes aligned.
